# EUROPÄISCHE PATENTSCHRIFT

(11) **EP 2 769 688 B1**
(45) Veröffentlichungstag und Bekanntmachung des Hinweises auf die Patenterteilung: **18.03.2015**
(21) Anmeldenummer: 14156478.1
(22) Anmeldetag: 25.02.2014
(51) Int. Cl.: A61B 17/122, A61B 17/128, A61B 17/29, A61B 17/22, A61B 17/00, A61B 17/12, A61B 17/28

(54) **Medizinisches Instrument mit einem korkenzieherartigen Verbindungselement**
Medical instrument having a corkscrew-like connecting element
Instrument médical doté d'un élément de liaison de type tire-bouchon

(30) Priorität: 26.02.2013 DE 102013101874
(43) Veröffentlichungstag der Anmeldung: 27.08.2014
(73) Patentinhaber: Karl Storz GmbH & Co. KG, 78532 Tuttlingen (DE)
(72) Erfinder: Besse, Régis, 78280 Guyancourt (FR); Thouément, Yann, 78690 Les Essarts le Roi (FR); Schneider, Sven, 78532 Tuttlingen (DE)
(74) Vertreter: Witte, Weller & Partner Patentanwälte mbB

(56) Entgegenhaltungen:
- WO-A1-93/09721
- WO-A2-95/11620
- US-A- 5 437 266
- US-A1- 2001 034 536
- US-A1- 2008 221 582
- US-A1- 2012 245 598
- US-A1- 2012 253 365

## Beschreibung

Die Erfindung betrifft ein medizinisches Instrument mit zwei Bauteilen, die über eine Haken/Ösen-Verbindung lösbar verbindbar sind, wobei ein erstes Bauteil eine Öse mit einem die Ösenöffnung vollständig umgebenden Rand aufweist, und ein zweites Bauteil ein Element aufweist, das in die Öse einbringbar ist und dabei eine zug-, schub- und drehfeste Verbindung zwischen den beiden Bauteilen schafft.

Ein derartiges medizinisches Instrument ist aus der US 5 749 881 A bekannt. Bei diesem medizinischen Instrument besteht das erste Bauteil aus einer Klammer, die zwei spreiz- bzw. schließbare Backen aufweist. An einem Ende ist eine Öse vorhanden. US 5 749 881 A offenbart ein medizinisches Instrument entsprechend dem Oberbegriff des Anspruchs 1.

Über diese Öse kann zeitweilig eine Verbindung mit einem zweiten Bauteil geschaffen werden, und zwar dadurch, dass dieses zweite Bauteil einen Haken aufweist, der in die Öse eingreifen kann. Durch diese Haken/Ösen-Verbindung wird eine zug-, schub- und drehfeste Verbindung zwischen den beiden Bauteilen geschaffen. Bei manchen Operationstechniken wird die Klammer im Körper gesetzt und klemmt dabei beispielsweise ein Gefäß oder ein Organ ab. Nach dem medizinischen Eingriff muss die Klammer wieder geborgen werden, was mit dem zweiten Bauteil erfolgt.

Ein ähnliches medizinisches Instrument ist aus der US 5 242 456 A1 bekannt. Auch hier ist das erste Bauteil als eine Klammer ausgebildet, die an ein Gewebe angesetzt werden kann und dort Bereiche klemmt oder festhält. Je nach operativem Eingriff kann das von der Klammer gehaltene Gewebeteil von den restlichen Bereichen abgetrennt werden, und anschließend kann die Klammer samt dem von dieser gehaltenen Gewebeteil über das zweite Bauteil nach Schließen der Haken/Ösen-Verbindung geborgen bzw. aus dem Körper entfernt werden. Diese Operationstechnik kann auch im Zusammenhang mit der minimalinvasiven Technologie eingesetzt werden.

Aus der WO 97/27808 A1 ist ein Instrument bekannt, das zur Entfernung von Blutpfropfen in Gefäßen eingesetzt wird. Dazu weist das Instrument an seinem distalen Ende einen schraubenlinienartig gewundenen Körper auf, der in den Blutpfropfen eingetrieben werden kann bzw. diesen einfängt, so dass dann der von dem schraubenlinienförmigen Körper gefangene Blutpfropfen aus dem Gefäß abgezogen werden kann.

Nachteilig an der eingangs genannten Haken/Ösen-Verbindung ist die Gefahr, dass sich diese Verbindung bei den Manipulationen löst. Der in dem eingangs genannten Dokument eingesetzte Haken ist etwa rechtwinklig aus einer Längsachse abgebogen und wird von einer Seite her in die Öse eingeschoben. Durch die entgegengesetzte Bewegung tritt aber der Haken wieder aus der Öse aus. Dadurch ist zwar ein einfaches Lösen dieser Haken/Ösen-Verbindung möglich, es besteht aber die Gefahr, dass ein solches Lösen ungewollt auch bei der Handhabung erfolgt.

Es wurde daher in Weiterentwicklungen versucht, die Haken stärker zu krümmen. Um jedoch die Haken überhaupt in die Öse einführen zu können, müssen diese an einem Bereich offen sein, was dann systemimmanent die Gefahr beinhaltet, dass über die entgegengesetzte Bewegung der Haken ungewollt wieder aus der Öse austritt.

Überlegungen, die Haken als Karabinerhaken oder als eine Art Rohrhaken auszubilden, können zwar das Problem des ungewollten Lösens der Haken/Ösen-Verbindung lösen. Nachteilig ist hierbei jedoch, dass bei einem solchen Karabinerhaken der Verschluss seitlich eingedrückt werden muss, damit der Karabiner wieder aus der Öse austreten kann.

Wie eingangs erwähnt, werden solche medizinischen Instrumente im Körper eingesetzt, so dass es äußerst schwierig ist, einen entsprechenden Karabinerhaken bzw. dessen Verschluss im Körper zu öffnen. Bei der minimalinvasiven Chirurgie sind die Instrumente meist in rohrförmigen Hohlschäften aufgenommen. Der Zugang zu einem seitlichen Verschluss eines Karabinerhakens ist dort kaum möglich.

Es ist daher Aufgabe der vorliegenden Erfindung, eine Haken/Ösen-Verbindung zwischen zwei Bauteilen zu schaffen, die zum einen einfach zu schließen ist und bei den üblichen Handhabungsmaßnahmen sicherstellt, dass sich diese Verbindung nicht versehentlich löst.

Erfindungsgemäß wird die Aufgabe dadurch gelöst, dass sich vom zweiten Bauteil in Richtung dessen Längsachse ein korkenzieherartig gewundener Verbindungskörper weg erstreckt, dessen Windungen sich in einem Abstand um die Längsachse winden und deren Ganghöhe zumindest der Stärke eines Randabschnittes des Randes der Öse entspricht.

Diese Maßnahme hat den Vorteil, dass die Verbindung zwischen den Bauteilen dadurch bewerkstelligt wird, dass der korkenzieherartig gewundene Verbindungskörper über dessen freies Ende in die Öse eingefädelt wird und durch Drehen des Verbindungskörpers sich mehr oder weniger Windungen in die Öse hinein oder hindurch winden. Dadurch, dass die Ganghöhe der Windungen zumindest der Stärke eines Randabschnittes entspricht, ist dieser dann spätestens nach Eindrehen einer ganzen Windung zwischen zwei Windungen gefangen aufgenommen. Schon in diesem Zustand ist eine Verbindung geschaffen, die zug- und schubfest ist und die, wenn die beiden Bauteile nicht zueinander verdreht werden, auch nicht versehentlich gelöst werden kann. Sind so viele Windungen in die Öse eingedreht, dass diese den gesamten Innenraum der Öse überspannen, führt ein weiteres Drehen des gewundenen Verbindungskörpers dazu, dass sich dann auch das erste Bauteil mit dem zweiten Bauteil dreht. Somit ist auch eine drehfeste Verbindung gegeben, allerdings nur in einer Richtung. Die Öse ist vollumfänglich mit Material umgeben und ist meist aus diesem Material herausgearbeitet. Ein Abschnitt des Randes, der die Öse umgibt, ist so ausgebildet, dass die Windungen sich um diesen Abschnitt in die Ösenöffnung hinein winden können.

Das Lösen der Verbindung wird einfach dadurch bewerkstelligt, dass der korkenzieherartig gewundene Verbindungskörper in die entgegengesetzte Richtung gedreht wird, wobei sich dann die Windungen wieder aus der Öse heraus winden.

Für die Handhabungsperson sind diese Vorgänge einfach und sicher durchführbar. Das Einfädeln wird regelmäßig unter Sicht durchgeführt werden. Der korkenzieherartige gewundene Verbindungskörper besitzt eine äußere Spitze, über die er an der Öse angesetzt bzw. diese Spitze in die Öse eingeschoben wird. Durch weiteres Drehen winden sich immer mehr Windungen in die Ösenöffnung hinein. Dies setzt sich so lange fort, bis die Windungen die gesamte Öffnung überspannen, danach kann der Verbindungskörper nicht mehr weiter eingedreht werden. Diesen Widerstand spürt die Handhabungsperson und weiß dann, dass die Endstellung erreicht worden ist. Dazu ist keine Sichtkontrolle notwendig. In diesem Zustand kann nun der Zusammenbau aus den beiden Bauteilen linear hin und her verschoben werden und auch in eine Richtung gedreht werden.

Soll die Verbindung gelöst werden, muss lediglich das zweite Bauteil in entgegengesetzter Richtung gedreht werden, so dass sich dann die Windungen aus der Öse wieder heraus winden.

Daher eignet sich diese Verbindung zwischen den Bauteilen insbesondere für die minimalinvasive Chirurgie, in der sich solche Vorgänge und Handhabungen in einem Hohlkörper oder in relativ engen Körperhöhlen abspielen.

Soll, wie eingangs erwähnt, eine bereits im Körper gesetzte Klammer geborgen werden, muss lediglich die distale äußere Spitze des gewundenen Verbindungskörpers an der Öse angesetzt werden, und dann kann der Körper eingedreht werden. Soll die Klammer durch Ziehen geborgen oder Gewebe retrahiert werden, reicht es schon aus, wenn eine oder zwei Windungen in die Öse eingedreht werden, denn dadurch ist diese Verbindung bereits sicher zugfest.

In einer weiteren Ausgestaltung der Erfindung endet der Verbindungskörper in einer äußeren Spitze, die etwa quer zur Längsachse verläuft.

Diese Maßnahme hat den Vorteil, dass das Einlaufen der Windungen in die Öse besonders zielgerichtet und sicher ablaufen kann. Die Spitze, die sich etwa quer zur Längsachse erstreckt, kann dann entsprechend seitlich an die Öse herangeführt und in die Ösenöffnung eingefädelt werden. Dies benötigt wenig Raum und kann daher sogar in einem Hohlschaft eines minimalinvasiven Instrumentes durchgeführt werden.

In einer weiteren Ausgestaltung beträgt der Gangwinkel der Windungen weniger als 45°.

In weiteren Ausgestaltungen der Erfindung liegt der Gangwinkel im Bereich zwischen 5° und 35°, höchst vorteilhaft im Bereich zwischen 10° und 20°.

Je geringer der Gangwinkel ist, desto weniger erheben sich die Windungen aus einer Ebene quer zur Längsachse. Bei sehr geringem Gangwinkel reicht es dann bereits aus, wenn eine komplette Windung in die Öse eingedreht ist, um schon eine sichere zug- und schubfeste Verbindung herzustellen.

Hat die Öse eine sehr kleine Öffnung, die nicht wesentlich größer ist als der Außendurchmesser des Drahtkörpers, der den Verbindungskörper aufbaut, benötigt man zwar eine gewisse Aufmerksamkeit, um die Spitze in eine solche kleine Öse einzufäden, allerdings kann hier schon eine halbe Drehung bei kleinem Gangwinkel ausreichen, um eine zug-, schub- und drehfeste Verbindung zu schaffen.

Ist die Ösenöffnung größer ausgebildet, ist es einfacher, die Spitze des Verbindungskörpers in die Öse einzufädeln, dann haben aber mehrere Windungen in der Öse Platz.

Dies kann gezielt gewollt sein, denn auf diese Weise ist es möglich, den Verbindungskörper relativ zur Öse hin und her zu schwenken oder abzuwinkeln, was bei manchen Handhabungen erwünscht sein kann. Wenn man beispielsweise eine Öse einer Klammer nicht direkt in der Längserstreckung der Klammer anzielen kann sondern sich aufgrund der anatomischen Gegebenheiten nur seitlich der Klammer nähern kann, können die Windungen von der Seite her in die Öse eingefädelt werden und dann, beispielsweise nach Herstellen der Verbindung, durch Ziehen an dem zweiten Bauteil in einer geradlinigen Ausrichtung ausgerichtet werden. In dieser Ausrichtung können die beiden Bauteile beispielsweise in einen Hohlschaft eines minimalinvasiven Instrumentes eingezogen und so einfach aus dem Körper entfernt werden.

Somit eröffnet diese Verbindung sehr viele Möglichkeiten der Ausgestaltung, zum einen, um sich an die jeweiligen örtlichen Gegebenheiten anpassen zu können, und zum anderen, um diese Verbindung sicher zu machen.

In einer weiteren Ausgestaltung der Erfindung weisen die Windungen über einen Abschnitt der Längsachse gesehen jeweils denselben Abstand zur Längsachse auf.

Diese Maßnahme hat den Vorteil, dass die äußere Hüllfläche des Verbindungskörpers zylindrisch ist, so dass ein Führen beim Hin-und-Her-Bewegen an der Innenwand eines rohrförmigen Hohlschaftes sehr gut möglich ist.

In einer weiteren Ausgestaltung der Erfindung sind die Außendurchmesser des ersten Bauteils und des zweiten Bauteils derart aufeinander abgestimmt, dass in verbundenem Zustand beide Bauteile in einen für die minimalinvasive Chirurgie geeigneten Hohlschaft aufnehmbar sind.

Wie bereits zuvor angedeutet, erlaubt diese Bemaßung einen Einsatz in der minimalinvasiven Chirurgie.

In einer weiteren Ausgestaltung der Erfindung sind die Windungen aus einem Draht mit gleichbleibendem Materialdurchmesser aufgebaut.

Das Drahtmaterial erlaubt zunächst einmal eine einfache Herstellung des korkenzieherartig gewundenen Verbindungskörpers durch einen Wickelvorgang um einen Kern. Der gleichbleibende Materialdurchmesser sorgt dafür, dass ein über seine gesamte Länge gleichartig stabiler Verbindungskörper entsteht.

In einer weiteren Ausgestaltung der Erfindung weist das erste Bauteil an einem Ende eine Klammer auf, die spreizbare Backen hat, während am anderen Ende die Öse vorhanden ist.

Wie bereits erwähnt, finden solche Bauteile häufig Einsatz bei chirurgischen Eingriffen, und derartige Klammern müssen bewegt, sprich gespreizt und geöffnet, werden und auch wieder aus dem Körper geborgen werden können.

In einer weiteren Ausgestaltung der Erfindung schließen sich die Backen beim Einziehen des ersten Bauteils in einen Hohlschaft, und das erste Bauteil ist durch das zweite Bauteil im Hohlschaft bewegbar.

Durch die Zugfestigkeit der Verbindung kann über das zweite Bauteil, aufgrund der Verbindung mit dem korkenzieherartig gewundenen Verbindungskörper, das erste Bauteil in einen Hohlschaft eingezogen werden, wodurch sich die über den Durchmesser des Hohlschafts gespreizten Backen schließen. Beispielsweise kann in diesem Zustand dieser Zusammenbau minimalinvasiv in einen Körper eingebracht werden. Danach kann das zweite Bauteil das erste Bauteil distal über den Hohlschaft hinaus schieben, wobei sich dann die Backen wieder spreizen und an ein Gefäß oder ein zu ergreifendes Gewebeteil angelegt werden können. Wird dieser Zusammenbau dann wieder in die entgegengesetzte Richtung bewegt, also in den Hohlschaft eingezogen, schließen sich wieder die Backen. Sollen beispielsweise die Backen für einen längeren Zeitraum während eines Eingriffs in diesem Zustand verbleiben, kann der korkenzieherartig gewundene Verbindungskörper aus der Öse ausgedreht und abgezogen werden. Zu einem späteren Zeitpunkt kann die Öse dann wieder eingeführt und die Verbindung wieder geschlossen werden, beispielsweise um die Klemme zu bergen.

In einer weiteren Ausgestaltung der Erfindung ist die Öse zumindest über deren halben äußeren Umfang von dem Randabschnitt umgeben.

Die Windungen müssen in die Öse eintreten können, so dass eine gewisse Abstimmung zwischen der Ösengeometrie und dem korkenzieherartig gewundenen Verbindungskörper vorhanden sein muss. Dazu ist es aber ausreichend, dass dieser Randabschnitt die Öse um deren halben äußeren Umfang umgibt, denn dieser Bereich ist in der Regel ausreichend, um die Windungen anzusetzen und in die Öse einzufädeln. Das heißt, am gegenüberliegenden Ende kann der Rand dann beispielsweise wesentlich dicker sein, beispielsweise ein stabförmiger Körper, an dessen Endseite die Öse herausgearbeitet ist.

In einer weiteren Ausgestaltung ist der Rand der Öse verschieden dick bzw. stark ausgebildet.

Dann kann ein Eindrehen der Windungen nur an bestimmten, dafür vorgesehenen dünnen Randabschnittstellen erfolgen.

In einer weiteren Ausgestaltung der Erfindung ist der äußere umfängliche Randabschnitt als U-förmiger Bügel ausgebildet.

Diese Maßnahme hat den Vorteil, dass dadurch eine ovalförmige, beispielsweise flachovalförmige, Ösenöffnung einer bestimmten Länge vorhanden ist. Dann kann der korkenzieherartig gewundene Verbindungskörper entsprechend auf die Geometrie abgestimmt seitlich angesetzt werden, und eine bestimmte Anzahl an Windungen kann eingedreht werden. Wie zuvor erwähnt, besteht ein relativ hoher Freiheitsgrad, um den ein in die Öse eingedrehter Verbindungskörper hin und her verschwenkt werden kann. Dies kann dazu ausgenützt werden, die Verbindung bzw. den U-förmigen Bügel als Flacheisen auszubilden, so dass dann bei entsprechender Anwinklung sogar der Zusammenbau in entsprechend verschwenktem Zustand in der Richtung gedreht werden kann, in der sich normalerweise die Windungen herausdrehen würden. Mit anderen Worten ausgedrückt, kann der in einige Windungen eingetretene Verbindungskörper durch entsprechendes Verschwenken so verkantet werden, dass der Zusammenbau auch in der Richtung gedreht werden kann, in der eigentlich der Drahtkörper ausgedreht werden kann. Durch das Verklemmen kann ein Ausdrehen aber erst dann erfolgen, wenn der Verbindungskörper dann wieder entsprechend ausgerichtet ist.

Dadurch ist es möglich, die Verbindung in beiden Richtungen drehfest zu bewerkstelligen.

In einer weiteren Ausgestaltung ist die Größe der Ösenöffnung derart gewählt, dass jeweils nur eine oder wenige Windungen einfädelbar sind.

Dadurch wird eine sichere Verbindung geschaffen, die jedoch einen hohen Grad an Abwicklungs- und Verschwenkungsmöglichkeiten der verbundenen Bauteile zueinander erlaubt.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: perspektivisch ein Ausführungsbeispiel eines ersten Bauteils des erfindungsgemäßen medizinischen Instruments mit der Öse, das als eine Klammer ausgebildet ist, die zwei Backen aufweist, die in Fig. 1 gespreizt sind,
- Fig. 2: eine Draufsicht auf die Klammer von Fig. 1 in einem Zustand, in dem die beiden Backen geschlossen sind,
- Fig. 3: eine Seitenansicht eines zweiten Bauteils des erfindungsgemäßen medizinischen Instruments mit einem korkenzieherartig gewundenen Verbindungskörper,
- Fig. 4: eine perspektivische Ansicht des Körpers von Fig. 3,
- Fig. 5: eine stirnseitige Draufsicht des Körpers von Fig. 3 und 4,
- Fig. 6: einen Zustand beim Herstellen der Verbindung zwischen den beiden Bauteilen, wobei gerade die Spitze des Verbindungskörpers des zweiten Bauteils in die Öse des ersten Bauteils eingedreht wird,
- Fig. 7: eine Darstellung, nachdem mehrere Windungen eingedreht worden sind,
- Fig. 8: eine Draufsicht auf den Zusammenbau aus den beiden Bauteilen mit in der Bauteilebene unter einem rechten Winkel zueinander stehenden Bauteilen,
- Fig. 9: eine Seitenansicht der medizinischen Vorrichtung mit aus der Bauteilebene heraus verschwenktem zweiten Bauteil,
- Fig. 10: eine teilweise geschnittene Seitenansicht einer erfindungsgemäßen Vorrichtung, die für einen minimalinvasiven Eingriff in einem Hohlschaft aufgenommen ist,
- Fig. 11: ein Bauteil mit einer Öse mit einer kleinen Ösenöffnung,
- Fig. 12: ein Bauteil mit einer Öse, deren Rand unterschiedlich dick bzw. stark ausgebildet ist,
- Fig. 13: schematisch ein praktischer Einsatz des Instrumentes vor dem Verbinden der beiden Bauteile, und
- Fig. 14: eine der Fig. 12 entsprechende Darstellung nach Verbinden der beiden Bauteile.

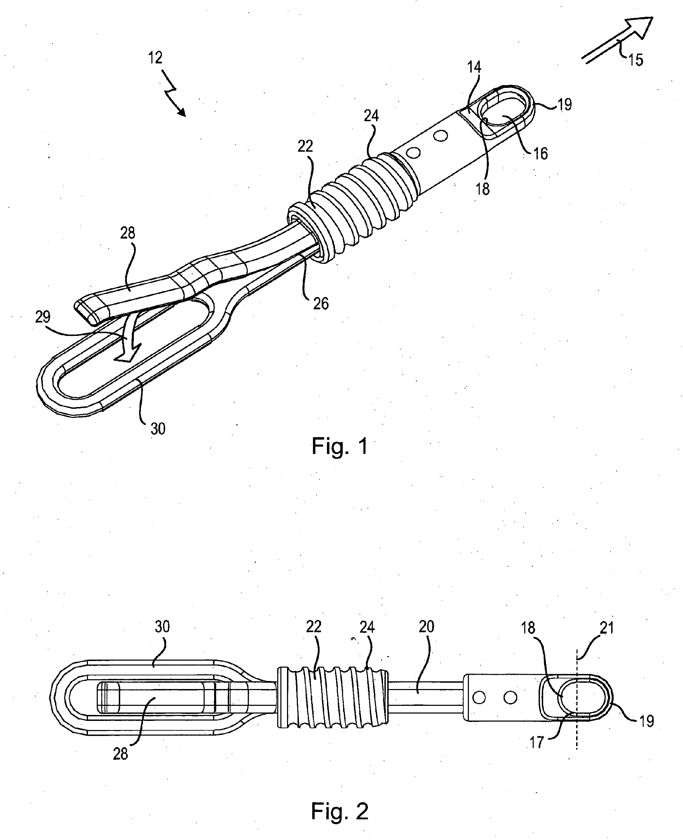

In den Fig. 1 und 2 ist ein erstes Bauteil 12 des medizinischen Instrumentes dargestellt.

Das erste Bauteil 12 weist an einem Ende eine Öse 14 auf, die eine Ösenöffnung 16 aufweist.

Wie insbesondere aus Fig. 2 zu ersehen, besteht die Ösenöffnung 16 aus einem länglichen Flachoval 17. Die Ösenöffnung 16 ist vollumfänglich von einem Rand 18 umgeben. Zumindest an ihrem äußeren Ende ist die Ösenöffnung 16 von einem Randabschnitt 19 umgeben, der ausgehend von einer gedachten gestrichelten Linie 21 in Fig. 2, als U-förmiger Bügel mit geradlinigen Schenkeln ausgebildet ist.

Die Öse 14 geht in einen stabförmigen Körper 20 über, auf dem verschieblich eine Hülse 22 mit einem Außengewinde 24 aufgenommen ist.

An dem der Öse 14 gegenüberliegenden Ende ist das erste Bauteil 12 als Klammer 26 aufgebaut, die zwei Backen 28 und 30 aufweist.

Dabei erstreckt sich die Backe 30 in einer Mittellängsebene des ersten Bauteils 12, von der die zweite Backe 28 hochsteht.

Die Backe 28 ist dabei gegenüber der Backe 30 so vorgespannt, dass sie, in der Stellung von Fig. 1, von der Backe 30 hochsteht. Wird an der Öse 14 in einer Richtung gezogen, wie das in Fig. 1 durch den Pfeil 15 dargestellt ist, und ist die Hülse 22 ortsfest in ein weiteres Bauteil eingeschraubt, so werden die beiden Backen 28 und 30 der Klammer 26 in die Hülse 22 eingezogen. Dabei drückt die Hülse 22 die hochstehende Backe 28 nach unten, wie das in Fig. 1 durch einen Pfeil 29 dargestellt ist. Dadurch wird dann ein zwischen den Backen 28 und 30 vorhandenes Teil, beispielsweise ein Gewebestück oder eine Ader, gehalten oder geklemmt.

Es ist ersichtlich, dass die Backe 30 als Hohloval ausgebildet ist, so dass die laschenförmige Backe 28 von einer Richtung her in das Hohloval eindringen und dadurch beispielsweise eine Ader oder dergleichen nicht nur festhalten, sondern auch gleich abklemmen kann.

Die hier beschriebene Ausgestaltung der Klammer 26 ist nur ein Beispiel. Es sind auch Klammern einsetzbar, die zwei bewegliche Backen aufweisen. Es sind auch Klammern einsetzbar, die in eine schließende Stellung vorgespannt sind. Diese klemmen ein Gewebe, wenn diese an einem Gewebe abgesetzt sind. Je nach Ausgestaltung öffnen diese bei der Handhabung oder können samt dem ergriffenen Gewebe bewegt oder aus dem Körper geborgen werden.

Derartige Bauteile 12 müssen also zu deren Manipulation hin und her bewegt werden, wozu sie mit einem weiteren Bauteil in Verbindung gesetzt werden, das über die Öse 14 eine Verbindung darstellt.

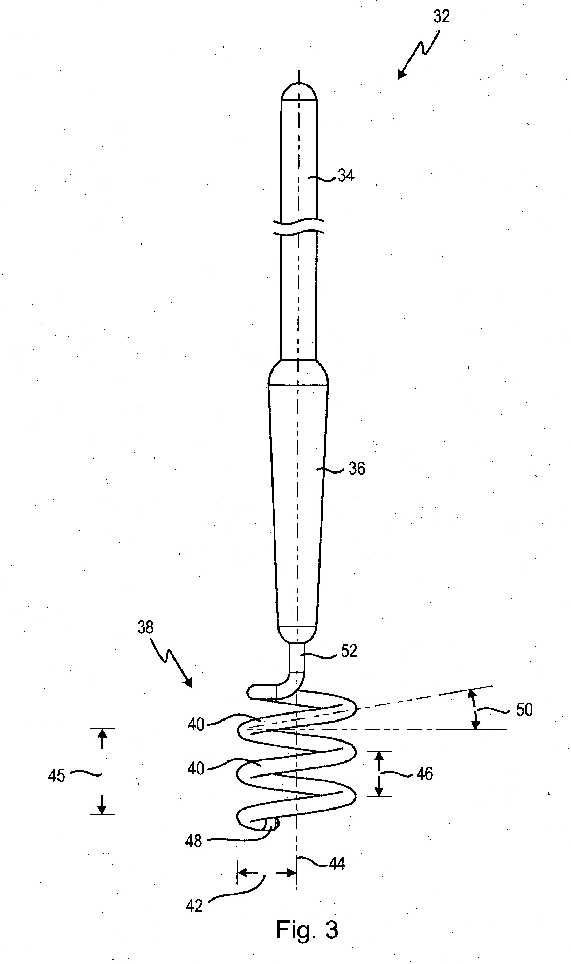

In den Fig. 3 bis 5 ist ein solches zweites Bauteil 32 dargestellt.

Das zweite Bauteil 32 weist einen langerstreckten stabförmigen Schaft 34 auf, der an einem Ende in einen etwas erweiterten, konisch zulaufenden Schaftabschnitt 36 übergeht.

Vom äußeren Ende des Schaftabschnittes 36 steht ein Verbindungskörper 38 vor, der dazu dient, eine Verbindung mit der zuvor beschriebenen Öse 14 des ersten Bauteils 12 herzustellen.

Der Verbindungskörper 38 besteht aus einem korkenzieherartig gewundenen Körper, der mehrere Windungen 40 aufweist. Die Windungen 40 sind nun so ausgestaltet, dass sie sich in einem Abstand 42 um eine Mittellängsachse 44 des zweiten Bauteils 32 winden. Zumindest über einen Abschnitt 45 weisen die Windungen 40 diesen gleichen Abstand 42 auf, so dass der Verbindungskörper 38, wenn man sich eine äußere Hüllfläche um die Windungen 40 vorstellt, zumindest in dem Abschnitt 45 eine zylindrische äußere Hüllkontur aufweist.

Die Ganghöhe 46 der Windungen 40 ist so gewählt, dass sie zumindest der Stärke des Randabschnittes 19 der zuvor beschriebenen Öse 14 entspricht.

Der Gangwinkel 50 beträgt etwa 10°.

Der Verbindungskörper 38 besteht aus einem metallischen Draht 52, der ausgehend von dem äußeren Ende des Schaftabschnittes 36 zu diesem schraubenlinienförmigen Verbindungskörper 38 geformt ist.

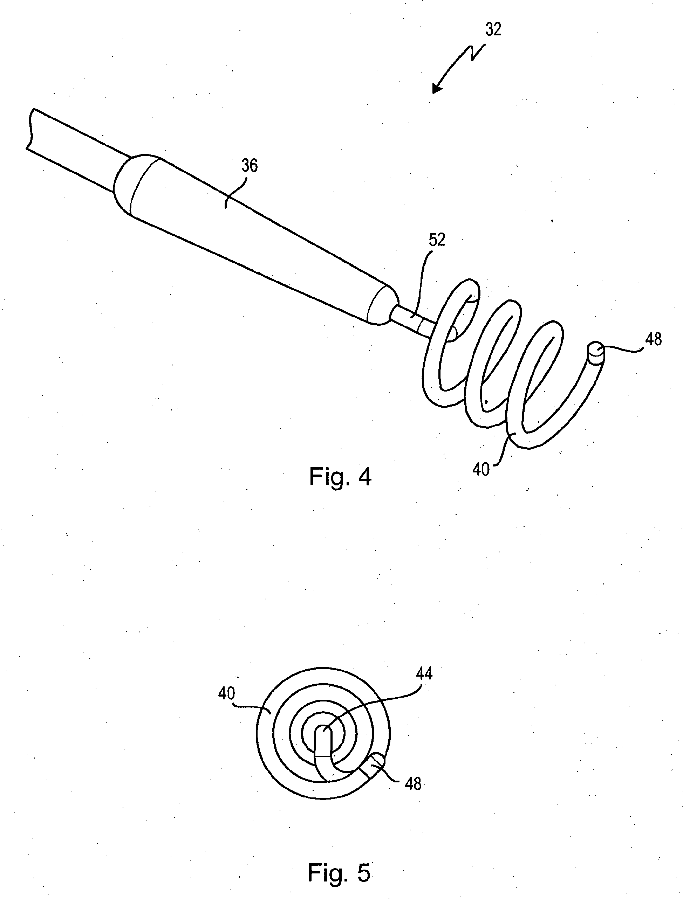

Am äußeren Ende endet der Verbindungskörper 38 in einer Spitze 48, die sich in einer Ebene erstreckt, wie das insbesondere aus Fig. 4 und 5 ersichtlich ist, die in etwa quer zur Mittellängsachse 44 verläuft. Die Spitze 48 stellt ein abgerundetes Ende des Drahtes 52 dar.

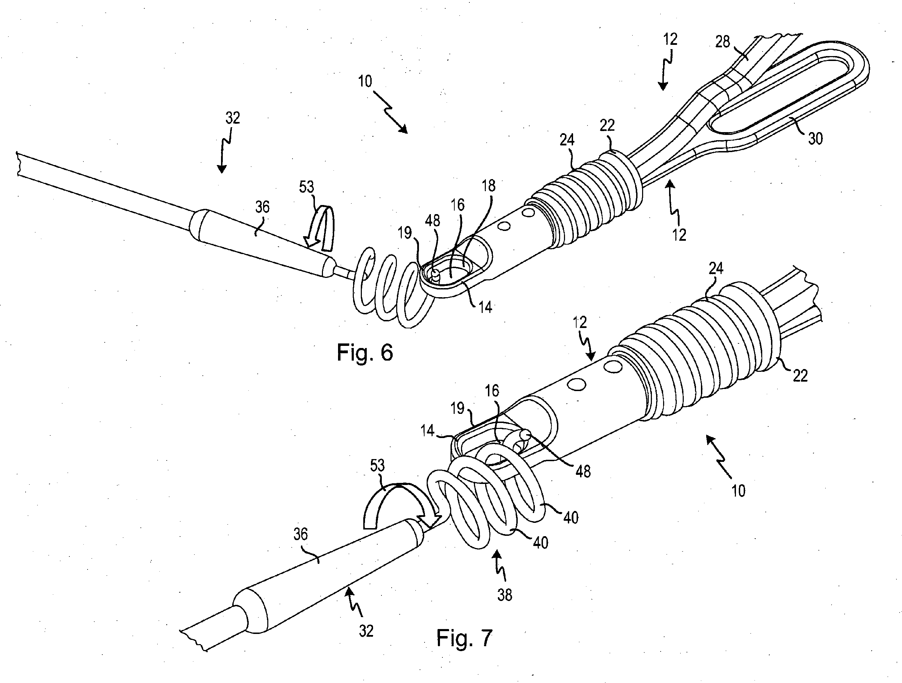

In Fig. 6 und 7 ist nunmehr dargestellt, wie die Verbindung zwischen der Öse 14 des ersten Bauteils 12 und dem Verbindungskörper 38 des zweiten Bauteils 32 zu dem medizinischen Instrument 10 hergestellt wird.

Aus Fig. 6 ist ersichtlich, dass die Spitze 48 des Verbindungskörpers 38 in die Ösenöffnung 16 eingeschoben wird und dass dann das zweite Bauteil 32 so gedreht wird, dass sich die Windungen 40 in die Öse hinein winden, wie das aus dem Übergang von Fig. 6 zu Fig. 7 ersichtlich ist.

Die schraubenlinienförmige Helix des Verbindungskörpers 38 ist somit eine rechtsläufige Helix, das heißt durch Drehen des zweiten Bauteils 32 im Uhrzeigersinn, wie das in Fig. 6 und 7 durch einen Pfeil 53 dargestellt ist, wird der Verbindungskörper 38 bzw. dessen Windungen 40 in die Ösenöffnung 16 eingedreht.

In Fig. 7 ist eine Situation dargestellt, bei der etwa eineinhalb Windungen 40 in die Ösenöffnung 16 eingedreht sind.

Dies reicht schon aus, um eine sichere zug- und schubfeste Verbindung zwischen dem ersten Bauteil 12 und dem zweiten Bauteil 32 herzustellen.

Ist der Verbindungskörper 38 so weit in die Öse 14 eingedreht, bis der Bereich der Spitze 48 an das, in Eindrehrichtung vorlaufende, Ende der Ösenöffnung 16 stößt, kann der Verbindungskörper 38 nicht mehr weiter eingedreht werden. Ein weiteres Drehen würde dann ein Drehen des zweiten Bauteils 32 verursachen, so dass spätestens in diesem Zustand dann auch zumindest in dieser Richtung eine drehfeste Verbindung hergestellt ist. Diese Drehrichtung kann beispielsweise dazu ausgenutzt werden, um das erste Bauteil 12 über dessen Hülse 22 in ein anderes Bauteil einzuschrauben.

Es ist auch möglich, gezielt so die Windungen 40 einzudrehen, dass jeweils nur eine einzige Windung 40 in der Ösenöffnung 16 eingedreht ist. Hat man die Spitze 48 einmal eingefädelt, kann man den Verbindungskörper 38 so lageverändern, dass bei einer weiteren Drehung die Spitze 48 an der Außenseite der Öse 14 vorbeiläuft. Dann ist jeweils nur eine einzige Windung 40 in der Ösenöffnung 16 eingefädelt. Das garantiert schon eine sichere Verbindung zwischen den Bauteilen, lässt aber erhebliche Verschwenk- bzw. Abwinklungsmöglichkeiten zwischen den verbundenen Bauteilen offen.

Zum Lösen der Verbindung zwischen erstem Bauteil 12 und zweitem Bauteil 32 wird dann das erste Bauteil 12 entgegen dem Uhrzeigersinn gedreht, so dass sich die Windungen 40 wieder aus der Ösenöffnung 16 der Öse 14 heraus winden.

Ist, wie zuvor im Zusammenhang mit Fig. 1 und 2 beschrieben, die Hülse 22 ortsfest montiert und wird, bei verbundenen Bauteilen 12 und 36 am ersten Bauteil 12, in der Darstellung von Fig. 7 nach links gezogen, wird die Klammer 26 in die Hülse eingezogen. Dadurch schließen die beiden Backen 28 und 30, so dass diese ein dazwischen vorhandenes Element klemmen oder festhalten können.

Aus den Darstellungen ist ersichtlich, dass der Durchmesser des Drahtes 52, der den Verbindungskörper 38 aufweist, wesentlich geringer ist als die Größe der Ösenöffnung 16. Dadurch ist es möglich, beispielsweise in dem in Fig. 7 dargestellten Verbindungszustand, die beiden so miteinander verbundenen Bauteile 12 und 36 relativ zueinander zu verschwenken.

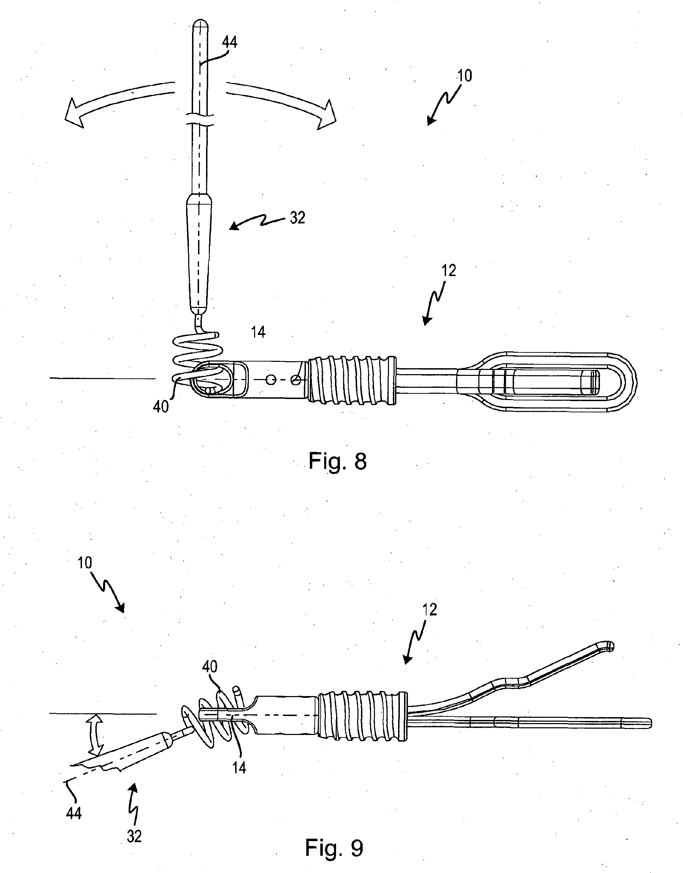

In Fig. 8 ist dargestellt, dass das zweite Bauteil 32 gegenüber der geradlinigen Erstreckung aus Fig. 7 etwa um 90° in der Geräteebene, die also hier der Papierebene entspricht, verschwenkt ist. Wie in Fig. 8 durch die beiden Pfeile gezeigt ist, kann das zweite Bauteil 32 über einen großen Bereich relativ zum ortsfest gedachten ersten Bauteil 12 verschwenkt werden. Das erhöht den Handhabungsspielraum. Es ist auch möglich, die Verbindung in einer solchen abgewinkelten Stellung zu bewerkstelligen, das heißt, die Windungen 40 können auch in dieser abgewinkelten Ausrichtung der beiden Bauteile 12 und 32 zueinander in die Öse 14 eingedreht oder auch wieder ausgedreht werden.

Dies ist beispielsweise im praktischen Einsatz dann erforderlich, wenn mehrere Klammern schon in einem Körper gesetzt und beispielsweise auf Klemmen oder Halten vorgespannt sind, und diese dann mit dem zweiten Bauteil 32 samt dem gehaltenen Gewebe geborgen werden sollen. Je nach den örtlichen Gegebenheiten kann es dann beispielsweise möglich sein, dass die Öse 14 von der Seite her angegangen werden kann, beispielsweise in der in Fig. 8 dargestellten Ausrichtung, und nicht in einer linearen Ausrichtung, wie in Fig. 7 dargestellt.

Dies ist beispielsweise schon aus Fig. 6 ersichtlich, das heißt, hier ist ersichtlich, dass man auch so angewinkelt die Verbindung herstellen kann.

in Fig. 9 ist noch ein weiterer Freiheitsgrad dargestellt, das heißt, das zweite Bauteil 32 kann nicht nur, wie in Fig. 8 dargestellt, in dieser Ebene verschwenkt werden, sondern auch aus dieser Ebene heraus, wie das in Fig. 9 durch den Doppelpfeil dargestellt ist.

Diese Verbindung über den Verbindungskörper 38 ist somit nicht nur einfach zu bewerkstelligen und zu lösen, sondern sie erlaubt der Handhabungsperson auch eine große Handlungs- und Manipulationsfreiheit.

Zuvor wurde beschrieben, dass die Öse an der Klammer vorhanden ist und der korkenzieherartige Körper am anderen Bauteil. Das kann auch umgekehrt der Fall sein.

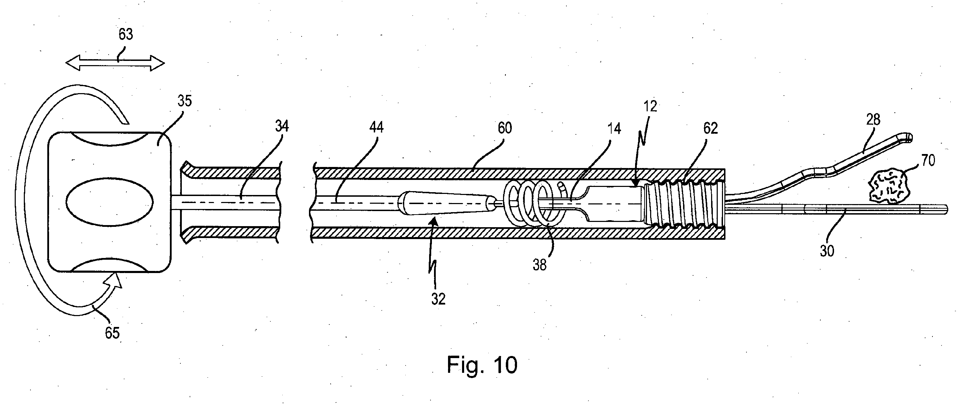

In Fig. 10 ist dargestellt, wie ein Zusammenbau aus erstem Bauteil 12 und zweitem Bauteil 32 in einem Hohlschaft 60 eines minimalinvasiven chirurgischen Instruments aufgenommen ist.

Der Hohlschaft 60 weist an einem Ende ein Innengewinde 62 auf, in das das Außengewinde 24 der Hülse 22 eindrehbar ist.

Vom distalen Ende steht die Klammer 26 vor, wobei hier die Backen 28 und 30 gespreizt sind.

Es ist dargestellt, dass die so gespreizten Backen 28 und 30 an ein Gewebeteil 70 herangeführt werden können, das durch die Klammer 26 erfasst werden soll. Das proximale Ende des Schaftes 34 des zweiten Bauteils 32 ist mit einem Griff 35 verbunden, der zur Manipulation bzw. Handhabung des zweiten Bauteils 32 dient. Wird beispielsweise der Griff 35 gedreht, wie das in Fig. 10 durch den Pfeil 65 dargestellt ist, dreht sich der Zusammenbau aus erstem Bauteil 12 und zweitem Bauteil 32 hier im Uhrzeigersinn, so dass beispielsweise durch diese Bewegung die Hülse 22 in das Innengewinde 62 des Hohlschafts 60 eingedreht werden kann.

Ist das erste Bauteil 12 aber schon vorher in dem Hohlschaft 60 entsprechend eingebaut, kann zum Herstellen der Verbindung das zweite Bauteil 32 von proximal nach distal in den Hohlschaft 60 eingeschoben werden, und durch Drehen kann dann die Verbindung zwischen den Windungen 40 des Verbindungskörpers 38 und der Öse 14 hergestellt werden.

Aus Fig. 10 ist ersichtlich, dass die zuvor dargestellte Ausgestaltung des Verbindungskörpers 38 derart ist, dass dessen äußere Konturlinie einem Zylinder entspricht, was für die Führung des Verbindungskörpers 38 in einem solchen Hohlschaft 60 sehr günstig ist.

Mit anderen Worten ausgedrückt, kann der Verbindungskörper 38 gut geführt und somit gezielt an die Öse 14 herangeführt werden und dann durch einfaches Drehen auch ohne sichtbare Kontrolle in die Ösenöffnung 16 eingedreht werden. Dies erleichtert die Handhabung in diesem Falle besonders.

In dem in Fig. 10 dargestellten montierten Zustand kann nun beispielsweise durch Bewegen des Zusammenbaus von distal nach proximal, wie das durch den Pfeil 63 dargestellt ist, die Klammer 26 durch die fest montierte Hülse 22 hindurch nach proximal gezogen werden, wie dies aus dem Übergang von Fig. 1 zu Fig. 2 ersichtlich ist. Dabei schließen die Backen 28 und 30 und halten dann das Gewebe 70 fest und sicher zwischen diesen. Je nach Ausgestaltung der Backen 28 und 30 und der Größenverhältnisse im Hohlschaft 60 und im Inneren der Hülse 22 können die Backen 28 und 30 vollständig in den Hohlschaft 60 eingezogen werden, beispielsweise, wenn diese schon vormontiert minimalinvasiv in dem Hohlschaft 60 in einen Körper eingeführt werden sollen. Dann sind diese beiden Backen 28 und 30 im Inneren des Hohlschafts 60 geschützt aufgenommen und können im Körper nach distal ausgeschoben werden. Dabei spreizt die Backe 28, und es kann dann ein Gewebestück 70 dazwischen gebracht und durch Einziehen der Backen 28 festgeklemmt werden.

Zuvor wurden ganz bestimmte Anwendungen beschrieben, es ist aber einleuchtend, dass eine solche Verbindung zwischen einem ersten Bauteil 12 und einem zweiten Bauteil 32 für andere Anwendungen eingesetzt werden kann. Es kommt eben darauf an, dass das eine erste Bauteil 12 eine Öse 14 aufweist, in die der schraubenlinienförmig oder korkenzieherartig gewundene Drahtkörper des Verbindungskörpers 38 des zweiten Bauteils 32 eingedreht werden kann.

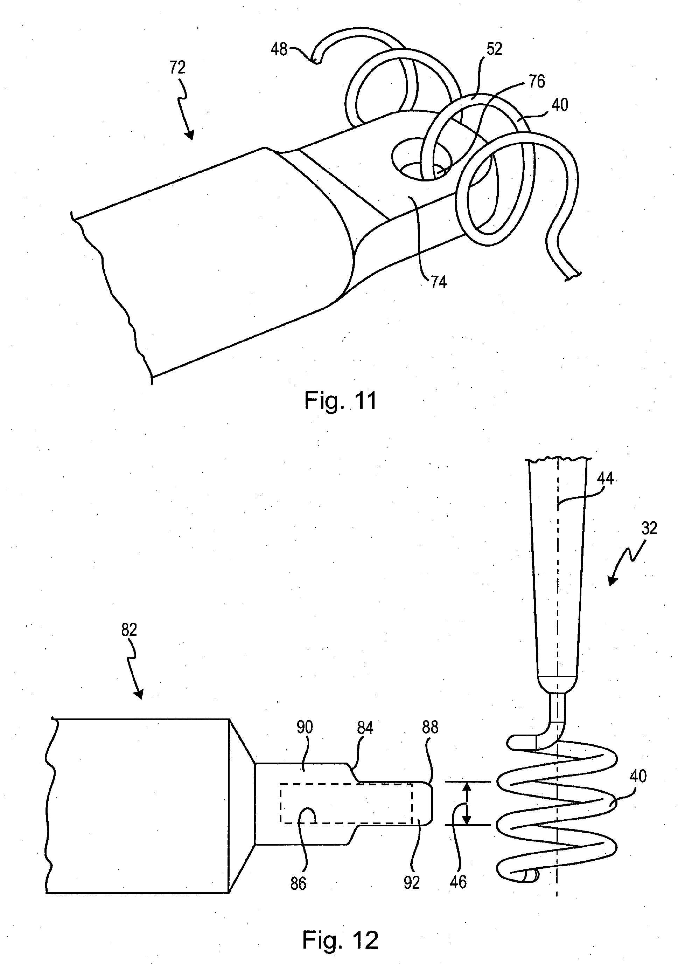

In Fig. 11 ist ein weiteres Ausführungsbeispiel eines Bauteils 72 dargestellt, das an seinem äußeren Ende eine Öse 74 mit einer Ösenöffnung 76 aufweist.

Gegenüber dem zuvor in Zusammenhang mit Fig. 1 und 2 dargestellten Ausführungsbeispiel ist die Ösenöffnung 76 relativ klein. Die Ösenöffnung 76 ist nur unwesentlich größer als der Durchmesser des Drahtes 52 der Windungen 40 des weiteren Bauteils. Dadurch befindet sich jeweils nur eine einzige Windung 40 in der Ösenöffnung 76. Diese Verbindung ist schon ausreichend sicher, um ein versehentliches Lösen zu verhindern. Gleichzeitig bestehen sehr große Freiheitsgrade in dem Abwinkeln und Verschwenken des Bauteils, das die Windungen 40 aufweist, gegenüber dem Bauteil, das diese Öse 74 mit der kleinen Ösenöffnung 76 aufweist. Um die Windungen 40 einzufädeln, muss dann deren Spitze 48 exakt an der kleinen Ösenöffnung 76 angesetzt werden, was üblicherweise unter Sichtkontrolle erfolgt.

In Fig. 12 ist ein weiteres Ausführungsbeispiel eines Bauteils 82 mit einer Öse 84 beschrieben, in der wieder eine relativ große langlochförmige Ösenöffnung 86 enthalten ist, wie das in Fig. 1 durch die Ösenöffnung in Form des Langovals 17 verwirklicht ist.

Hier ist der Rand 88, der die Ösenöffnung 86 umgibt, abgestuft.

Ein erster Abschnitt 90 weist eine solche Dicke oder Stärke auf, die größer ist als die Ganghöhe 46 der Windungen 40 des Bauteils 32, das in die Ösenöffnung 86 eingedreht werden soll.

Der Rand 88 weist einen äußeren zweiten Randabschnitt 92 auf, der dünner ist und dessen Stärke bzw. Dicke etwas geringer ist als die Ganghöhe 46 der Windungen 40.

Daher können die Windungen 40 des zweiten Bauteils 32 nur im Bereich des zweiten Randabschnitts 92 in die Ösenöffnung 86 eingedreht werden.

Das ist nur eine Möglichkeit eines abgestuften Randes, es kann auch umgekehrt sein, dass der innere Abschnitt dünner ist und der äußere breiter. Es kann auch sein, dass der Rand nur eine Kerbe aufweist, so dass dann die Windungen 40 nur im Bereich der Kerbe eingedreht werden können.

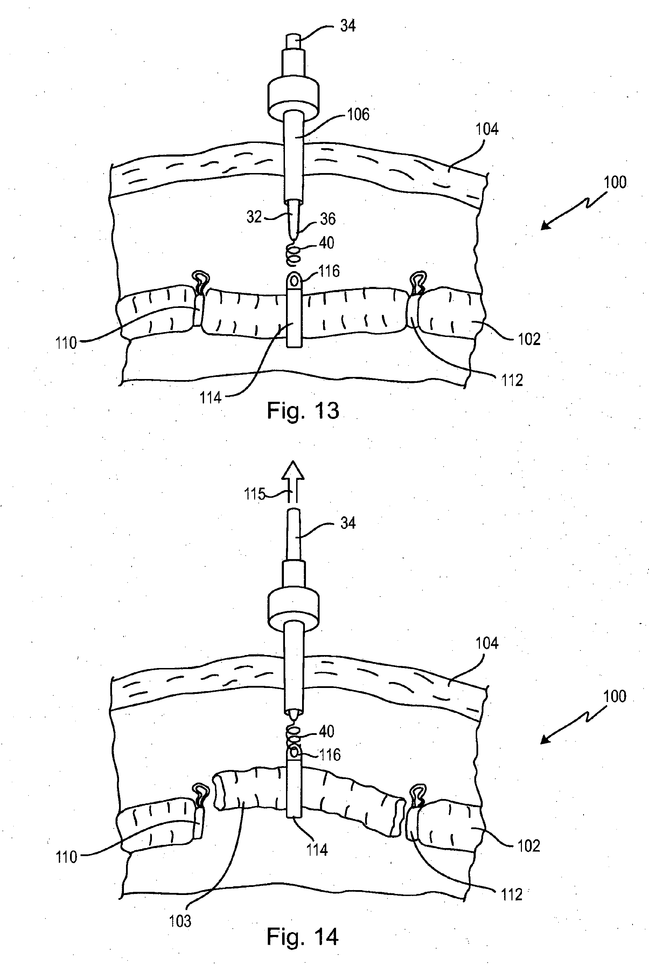

In den Fig. 13 und 14 ist ein Beispiel eines praktischen Einsatzes des erfindungsgemäßen medizinischen Instrumentes schematisch dargestellt.

Fig. 13 zeigt einen ausschnittsweisen Schnitt durch einen Körper 100, in dessen Inneren ein Gefäß 102 vorhanden ist. Das Gefäß 102 kann beispielsweise eine Ader oder ein Darmabschnitt sein.

In die Haut 104 des Körpers 100 ist eine Kanüle 106 eingesteckt, in der ein zweites Bauteil 32 aufgenommen ist. Dabei ragt der Schaftabschnitt 36 mit den Windungen 40 distalseitig über das Ende der Kanüle 106 hinaus.

Proximalseitig steht der Schaft 34 über.

Es ist ersichtlich, dass das Gefäß 102 von einer Klammer 114 ergriffen ist, von der eine Öse 116 absteht.

Die Klammer 114 ist eine auf Schließen vorgespannte Klammer und wurde zuvor an dem Gefäß 102 abgesetzt. Dadurch umgreift die Klammer das Gefäß 102 in diesem Bereich.

Aus Fig. 13 ist zu entnehmen, dass beidseits der Klammer 114 das Gefäß 102 durch Schlingen 112 eingeschnürt oder ggf. schon aufgetrennt ist.

In Fig. 14 ist nun eine Situation dargestellt, bei der das zweite Bauteil 32 so weit nach distal durch die Kanüle 106 verschoben worden ist, bis die Windungen 40 in die Öse 116 der Klammer 114 eingedreht werden konnten. Dies kann unter Sicht dadurch geschehen, dass durch die Haut 104 ein Beobachtungsinstrument, beispielsweise ein Endoskop, durchgeschoben ist.

Nach Schließen der Verbindung zwischen dem zweiten Bauteil 32 und der Klammer 114 durch Eindrehen der Windungen 40 in die Öse 116 kann der Schaft 34 des zweiten Bauteils 32, wie das in Fig. 14 durch einen Pfeil 115 dargestellt ist, nach proximal gezogen werden. Das von der Klammer 114 gehaltene Teilstück 103 des Gefäßes 102 wird dabei mit bewegt und kann von dem verbleibenden Gefäß 102 abgetrennt werden. In Fig. 14 ist eine Situation dargestellt, bei der dieses Teilstück 103 bereits abgetrennt ist.

Das so ergriffene Teilstück 102 kann nunmehr geborgen, beispielsweise aus dem Körper 110 abgezogen werden.

Danach können die beiden gegenüberstehenden Enden im Bereich der Schlingen 110, 112 wieder aneinandergefügt und miteinander verbunden werden.

## Patentansprüche

1. Medizinisches Instrument, mit zwei Bauteilen (12, 32, 72, 82), die über eine Haken/Ösen-Verbindung lösbar verbindbar sind,
wobei ein erstes Bauteil (12, 72, 82) eine Öse (14, 74, 84) mit einem die Ösenöffnung (16, 76, 86) vollständig umgebenden Rand (18, 88) aufweist,
und ein zweites Bauteil (32) ein Element aufweist, das in die Öse (14, 74, 84) einbringbar ist und dabei eine zug-, schub- und drehfeste Verbindung zwischen den beiden Bauteilen (12, 32, 72, 82) schafft,
**dadurch gekennzeichnet, dass** das Element des zweiten Bauteils (32) einen sich in Richtung der Längsachse (44) des zweiten Bauteils (32) korkenzieherartig gewundenen Verbindungskörper (38) aufweist, dessen Windungen (40) sich in einem Abstand (42) um die Längsachse (44) winden und deren Ganghöhe (46) zumindest der Stärke eines Randabschnittes (19, 92) des Randes (18, 88) der Öse (14, 74, 84) entspricht.

2. Medizinisches Instrument nach Anspruch 1, **dadurch gekennzeichnet, dass** der Verbindungskörper (38) in einer äußeren Spitze (48) endet, die etwa quer zur Längsachse (44) verläuft.

3. Medizinisches Instrument nach Anspruch 1 oder 2, **dadurch gekennzeichnet, dass** der Gangwinkel (50) der Windungen (40) kleiner als 45° ist.

4. Medizinisches Instrument nach Anspruch 3, **dadurch gekennzeichnet, dass** der Gangwinkel (50) zwischen 5° und 35° liegt.

5. Medizinisches Instrument nach Anspruch 3, **dadurch gekennzeichnet, dass** der Gangwinkel (50) zwischen 10° und 20° liegt.

6. Medizinisches Instrument nach einem der Ansprüche 1 bis 5, **dadurch gekennzeichnet, dass** die Windungen (40) über einen Abschnitt (45) der Längsachse (44) jeweils denselben Abstand (42) zur Längsachse (44) aufweisen.

7. Medizinisches Instrument nach einem der Ansprüche 1 bis 6, **dadurch gekennzeichnet, dass** die Außendurchmesser von erstem Bauteil (12) und zweitem Bauteil (32) derart aufeinander abgestimmt sind, dass in verbundenem Zustand beide Bauteile (12, 32) in einem für die minimalinvasive Chirurgie geeigneten Hohlschaft (60) aufnehmbar sind.

8. Medizinisches Instrument nach einem der Ansprüche 1 bis 7, **dadurch gekennzeichnet, dass** die Windungen (40) aus einem Draht (52) mit gleichbleibendem Materialdurchmesser aufgebaut sind.

9. Medizinisches Instrument nach einem der Ansprüche 1 bis 8, **dadurch gekennzeichnet, dass** das erste Bauteil (12) an einem Ende eine Klammer (26) aufweist, die spreizbare Backen (28, 30) hat und am anderen gegenüberliegenden Ende die Öse (14) aufweist.

10. Medizinisches Instrument nach Anspruch 9, **dadurch gekennzeichnet, dass** die Backen (28, 30) beim Einziehen in einen Hohlschaft (60) schließen, und dass das zweite Bauteil (32) im Hohlschaft (60) bewegbar ist.

11. Medizinisches Instrument nach einem der Ansprüche 1 bis 10, **dadurch gekennzeichnet, dass** die Öse (14) zumindest über deren halben äußeren Umfang von dem Randabschnitt (19) umgeben ist.

12. Medizinisches Instrument nach Anspruch 11, **dadurch gekennzeichnet, dass** der Randabschnitt (19) als U-förmiger Bügel ausgebildet ist.

13. Medizinisches Instrument nach einem der Ansprüche 1 bis 12, **dadurch gekennzeichnet, dass** die Größe der Ösenöffnung (76) derart gewählt ist, dass nur eine oder wenige Windungen (40) einfädelbar sind.

14. Medizinisches Instrument nach einem der Ansprüche 1 bis 13, **dadurch gekennzeichnet, dass** der Rand (88) nur in dem Randabschnitt (92) eine Stärke aufweist, die der Ganghöhe (46) entspricht, ansonsten aber stärker ausgebildet ist.

## Claims

1. Medical instrument with two component parts (12, 32, 72, 82) that are releasably connectable via a hook/eyelet connection, wherein a first component part (12, 72, 82) comprises an eyelet (14, 74, 84) having an edge (18, 88) that fully surrounds the eyelet opening (16, 76, 86), and a second component part (32) comprises an element that can be introduced into the eyelet (14, 74, 84) and in so doing creates a connection between the two component parts (12, 32, 72, 82), which is pull-tight, push-tight and rotation-tight, **characterized in that** the element of the second component part (32) comprises a connection body (38) which is wound in a corkscrew-like manner in the direction of the longitudinal axis (44) of the second component part (32), the turns (40) of the connection body winding around the longitudinal axis (44) at a distance (42), and the lead (46) of the turns corresponding at least to the thickness of an edge portion (19, 92) of the edge (18, 88) of the eyelet (14, 74, 84).

2. Medical instrument of Claim 1, **characterized in that** the connection body (38) ends in an outer tip (48) that runs approximately transversely to the longitudinal axis (44).

3. Medical instrument of Claims 1 or 2, **characterized in that** the helix angle (50) of the turns (40) is less than 45°.

4. Medical instrument of Claim 3, **characterized in that** the helix angle (50) is between 5° and 35°.

5. Medical instrument of Claim 3, **characterized in that** the helix angle (50) is between 10° and 20°.

6. Medical instrument of anyone of Claims 1 to 5, **characterized in that** the turns (40) are each arranged at the same distance (42) from the longitudinal axis (44) over a portion (45) of the longitudinal axis (44).

7. Medical instrument of anyone of Claims 1 to 6, **characterized in that** the outer diameters of the first component part (12) and second component part (32) are matched to one another in such a way that, in the connected state, both component parts (12, 32) can be received in a hollow shaft (60) suitable for minimally invasive surgery.

8. Medical instrument of anyone of Claims 1 to 7, **characterized in that** the turns (40) are formed from a wire (52) having constant material diameter.

9. Medical instrument of anyone of Claims 1 to 8, **characterized in that** the first component part (12), at one end, comprises a clamp (26) which has spreadable jaws (28, 30), and, at the opposite end, comprises the eyelet (14).

10. Medical instrument of Claim 9, **characterized in that** the jaws (28, 30) close when drawn into a hollow shaft (60), and **in that** the second component part (32) is movable in the hollow shaft (60).

11. Medical instrument of anyone of Claims 1 to 10, **characterized in that** the eyelet (14) is surrounded at least over half of its outer circumference by the edge portion (19).

12. Medical instrument of Claim 11, **characterized in that** the edge portion (19) is formed as a U-shaped shackle.

13. Medical instrument of anyone of Claims 1 to 12, **characterized in that** the size of the eyelet opening (76) is selected in such a way that only one turn or a few turns (40) can be threaded in.

14. Medical instrument of anyone of Claims 1 to 13, **characterized in that** the edge (88), only in the edge portion (92), has a thickness that corresponds to the lead (46), but otherwise is thicker.

## Revendications

1. Instrument médical, doté de deux composants (12, 32, 72, 82), qui peuvent être reliés de manière amovible par une liaison crochet/oeillet,
où un premier composant (12, 72, 82) comporte un oeillet (14, 74, 84) avec une bordure (18, 88) entourant entièrement l'ouverture d'oeillet (16, 76, 86),
et un deuxième composant (32) comporte un élément, qui peut être introduit dans l'oeillet (14, 74, 84) et créé de cette manière une liaison résistant à la traction, résistant à la poussée et résistant à la torsion entre les deux composants (12, 32, 72, 82),
**caractérisé en ce que** l'élément du deuxième composant (32) comporte un corps de liaison (38) s'enroulant en tire-bouchon dans la direction de l'axe longitudinal (44) du deuxième composant (32), dont les spires (40) s'enroulent selon une certaine distance (42) autour de l'axe longitudinal (44) et dont le pas (46) correspond au minimum à la hauteur d'une partie de bordure (19, 92) de la bordure (18, 88) de l'oeillet (14, 74, 84).

2. Instrument médical selon la revendication 1, **caractérisé en ce que** le corps de liaison (38) se termine par une pointe extérieure (48), qui s'étend sensiblement de manière transversale par rapport à l'axe longitudinal (44).

3. Instrument médical selon la revendication 1 ou 2, **caractérisé en ce que** l'angle d'enroulement (50) des spires (40) est inférieur à 45°.

4. Instrument médical selon la revendication 3, **caractérisé en ce que** l'angle d'enroulement (50) est compris entre 5° et 35°.

5. Instrument médical selon la revendication 3, **caractérisé en ce que** l'angle d'enroulement (50) est compris entre 10° et 20°.

6. Instrument médical selon l'une des revendications 1 à 5, **caractérisé en ce que** les spires (40) s'étendant sur une partie (45) de l'axe longitudinal (44) présentent chacune la même distance (42) par rapport à l'axe longitudinal (44).

7. Instrument médical selon l'une des revendications 1 à 6, **caractérisé en ce que** les diamètres extérieurs du premier composant (12) et du deuxième composant (32) concordent l'un avec l'autre de telle sorte que lorsqu'ils sont reliés, les deux composants (12, 32) peuvent être reçus dans une tige creuse (60) adaptée à la chirurgie mini-invasive.

8. Instrument médical selon l'une des revendications 1 à 7, **caractérisé en ce que** les spires (40) sont conçues à partir d'un fil (52) présentant un diamètre constant.

9. Instrument médical selon l'une des revendications 1 à 8, **caractérisé en ce que** le premier composant (12) comporte au niveau d'une première extrémité une attache (26), qui présente des mâchoires écartées (28, 30) et comporte l'oeillet (14) au niveau de l'autre extrémité opposée.

10. Instrument médical selon la revendication 9, **caractérisé en ce que** les mâchoires (28, 30) se ferment lors d'une insertion dans une tige creuse (60), et **en ce que** le deuxième composant (32) peut être déplacé dans la tige creuse (60).

11. Instrument médical selon l'une des revendications 1 à 10, **caractérisé en ce que** l'oeillet (14) est entouré sur au moins la moitié de sa circonférence extérieure de la partie de bordure (19).

12. Instrument médical selon la revendication 11, **caractérisé en ce que** la partie de bordure (19) se présente sous la forme d'une armature en U.

13. Instrument médical selon l'une des revendications 1 à 12, **caractérisé en ce que** la dimension de l'ouverture d'oeillet (76) est choisie de telle sorte qu'une seule spire ou plusieurs spires (40) peuvent être insérées.

14. Instrument médical selon l'une des revendications 1 à 13, **caractérisé en ce que** la bordure (88) présente une hauteur qui correspond au pas (46) seulement au niveau de la partie de bordure (92), mais présente ailleurs une hauteur plus importante.
